(19) **Europäisches Patentamt / European Patent Office / Office européen des brevets**

(11) **EP 3 273 850 B1**

(12) **EUROPEAN PATENT SPECIFICATION**

(45) Date of publication and mention of the grant of the patent:
**24.11.2021 Bulletin 2021/47**

(21) Application number: **16714251.2**

(22) Date of filing: **15.03.2016**

(51) Int Cl.:
*A61B 5/024* (2006.01)      *A61B 5/1455* (2006.01)
*H01L 33/44* (2010.01)      *H01L 33/50* (2010.01)

(86) International application number:
**PCT/EP2016/055482**

(87) International publication number:
**WO 2016/150749 (29.09.2016 Gazette 2016/39)**

(54) **OPTICAL VITAL SIGNS SENSOR**

OPTISCHER LEBENSZEICHENSENSOR

DÉTECTEUR OPTIQUE DE SIGNES VITAUX

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2015 EP 15160262**

(43) Date of publication of application:
**31.01.2018 Bulletin 2018/05**

(73) Proprietor: **Koninklijke Philips N.V.**
**5656 AG Eindhoven (NL)**

(72) Inventors:
• **PAULUSSEN, Elvira Johanna Maria**
**5656 AE Eindhoven (NL)**

• **VERMEULEN, Olaf Thomas Johan Antonie**
**5656 AE Eindhoven (NL)**

(74) Representative: **Philips Intellectual Property & Standards**
**High Tech Campus 5**
**5656 AE Eindhoven (NL)**

(56) References cited:
**EP-B1- 2 139 383        WO-A1-2014/136706**
**JP-A- 2001 025 462      US-A1- 2012 078 116**
**US-A1- 2014 243 648**

**Description**

FIELD OF THE INVENTION

The invention relates to an optical vital signs sensor for monitoring vital signs of a user.

BACKGROUND OF THE INVENTION

Optical heart rate sensors are well known to monitor or detect vital signs like a heart rate of a user. Such a heart rate sensor can be based on a photoplethysmograph (PPG) sensor and can be used to acquire a volumetric organ measurement. By means of pulse oximeters, changes in light absorption of a human skin is detected and based on these measurements a heart rate or other vital signs of a user can be determined. The PPG sensors comprise a light source like a light emitting diode (LED) which is emitting light into the skin of a user. The emitted light is scattered in the skin and is at least partially absorbed by the blood. Part of the light exits the skin and can be captured by a photodiode. The amount of light that is captured by the photo diode can be an indication of the blood volume inside the skin of a user. A PPG sensor can monitor the perfusion of blood in the dermis and subcutaneous tissue of the skin through an absorption measurement at a specific wave length. If the blood volume is changed due to the pulsating heart, the scattered light coming back from the skin of the user is also changing. Therefore, by monitoring the detected light signal by means of the photodiode, a pulse of a user in his skin and thus the heart rate can be determined. Furthermore, compounds of the blood like oxygenated or deoxygenated hemoglobin as well as oxygen saturation can be determined.

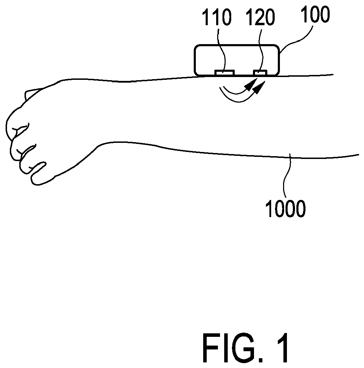

Fig. 1 shows a basic representation of an operational principle of a heart rate sensor. In Fig.1, a heart rate sensor is arranged on an arm of a user. The heart rate sensor 100 comprises a light source 110 and a photo detector 120. The light source 110 emits typically green light onto or in the skin 1000 of a user. Some of the light is reflected and the reflected light can be detected by the photo detector 120. Some light can be transmitted through tissue of the user and be detected by the photo detector 120. Based on the reflected or transmitted light, vital signs of a user like a heart rate can be determined.

WO 2006/110488 A2 shows a PPG sensor with coupling gel proximate to a light source of the PPG sensor.

US 2012/0078116 A1 discloses an optical vital signs sensor with a contact surface, a light source and a photo detector as well as a filter adapted to remove part of the light spectrum.

EP 2 139 383 B1 discloses an optical vital signs sensor with a light source, a photo detector and a filter for removing part of the lights spectrum.

JP 2001025462 A discloses an optical vital signs sensor with a light source, a photo detector and a filter in form of a coated acrylic board.

US 2014/0243648 A1 discloses an optical vital signs sensor with a light source, a photo detector and a colored converting plate.

SUMMARY OF THE INVENTION

It is an object of the invention to provide an optical vital signs sensor which is able to more efficiently detect vital signs of a user.

According to an aspect of the invention, an optical vital sensor according to claim 1 is provided.

The diffusion chamber has a recycling function, namely it is re-trying to convert the unconverted light.

According to an aspect of the invention, the color converting plate comprises a long-wave pass filter coating or film which is able to transmit light having a long wavelength while reflecting light having short wavelengths.

According to a further aspect of the invention, a method according to claim 6 is provided.

The vital signs sensor can be a LED based PPG sensor. The LED light penetrates the skin of the user and some of it can reach a photo detector. The output of the photo detector can be used to monitor a blood volume fraction and blood compounds like oxygenated and deoxygenated hemoglobin. In particular, the amount of absorption or reflectance of the light from the LED light source can be used to determine the heart rate as well as the blood volume fraction or blood compounds. The heart rate relates to the blood volume fraction. Furthermore, the PPG sensor is therefore an optical sensor allowing a non-invasive measurement of vital signs of a user.

These and other aspects will be apparent from and elucidated with reference to the embodiment(s) described hereinafter.

BRIEF DESCRIPTION OF THE DRAWINGS

In the following drawings:

Fig. 1 shows a basic representation of an operational principle of a vital sign monitoring system,
Fig. 2 shows a schematic representation of an optical vital signs sensor,
Fig. 3 shows a schematic representation of an optical vital signs sensor according to an aspect of the invention,
Fig. 4 shows a schematic representation of an optical vital signs sensor according to a further aspect of the invention,
Fig. 5 shows a graph indicating the function of the transmittance over the wavelength of the optical vital signs sensor according to Fig. 4,
Fig. 6 shows a basic representation of part of an optical vital signs sensor according to a further aspect of the invention,

Fig. 7 shows a basic representation of a part of an optical vital signs sensor according to a further aspect of the invention,

Fig. 8 shows a graph for illustrating a relative DC power and AC/DC signal of an optical vital signs sensor according to an aspect of the invention,

Fig. 9 shows a basic representation of a vital signs sensor, and

Fig. 10 shows a basic representation of a vital signs sensor.

## DETAILED DESCRIPTION

An optical vital signs sensor is provided which is based on a photoplethysmograph PPG sensor. Such a PPG sensor is depicted in Fig. 1. A light source 110 emits light onto or into the skin 1000 of a user and some of the light is reflected and this reflected light can be detected by a photo detector 120. The output of the photo detector can be analyzed to determine a heart rate or other vital signs of a user.

The output signal of the PPG sensor gives an indication on the blood movement in vessels of a user. The quality of the output signal of the PPG sensor can depend on the blood flow rate, skin morphology and skin temperature. In addition, optical losses in the PPG sensor may also have an influence on the quality of the output signal of the PPG sensor. The optical efficiency of the PPG sensor can depend on reflection losses when light penetrates from one media into another. Furthermore, a scattering of light at the surface of the skin of the user may also have an influence on the optical efficiency of the PPG sensor.

The PPG sensor or optical vital signs sensor can be implemented as a wearable device which can be arranged or attached to a skin of a user. The wearable device can be a wrist device (like a watch or smart watch). A device worn behind the ear of a user, e.g. like a hearing aid.

At least one of the light sources 110 can be implemented as a phosphor converted light emitting diode LED which comprises a color converting plate unit which is arranged at a contact surface of the sensor. The contact surface is that surface of the PPG sensor which is placed against the skin of a user. In other words, the color converting plate unit can be part of the contact surface and can thus be in direct contact with the skin of the user. The color converting plate unit can thus be arranged between the skin of the user and the light source or a light emitting diode LED in the light source. The color converting plate unit receives light and output light with a new emission spectrum. This can e.g. be performed by wavelength conversion through photo luminescence. The color converting plate can be thus implemented as wavelength conversion unit, wherein the wavelength conversion is based on photo luminescence.

An optical interface is provided between the light delivery system, namely the light source 110 and the skin 1000 of the user. This optical surface (e.g. in form of the color converting plate) is used to reduce reflectance losses and to increase the efficiency of the vital signs sensor.

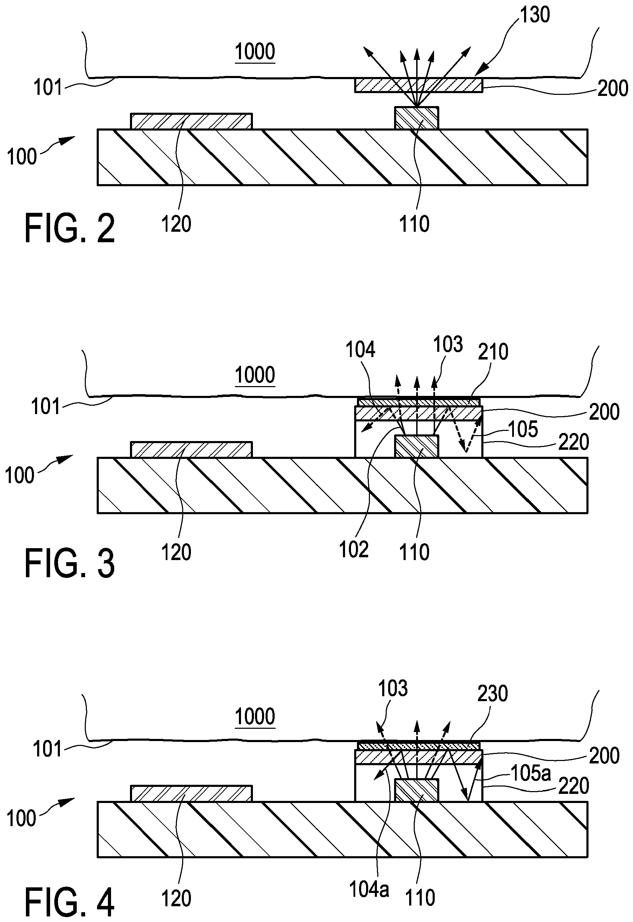

Fig. 2 shows a schematic representation of an optical vital signs sensor according to an aspect of the invention. The PPG sensor 100 comprises a contact surface 101 which is placed in direct contact to a skin 1000 of a user. Optionally, the converting plate can be directly mounted onto the light source area. Furthermore, the PPG sensor 100 also comprises at least one light source 110 as well as at least one photo detector unit 120. The at least one light source 110 emits light into the direction of the contact surface 101. Between the at least one light source 110 and the contact surface 101, a color converting plate unit 200 is provided. The color converting plate unit 200 can for example be implemented as a ceramic phosphor color converting plate. The at least one light source 110 can be implemented as a InGaN light emitting diode which is down-converted to for example yellow (having a wavelength of 570 nm) for example by means of the color conversion plate unit 200. As the color converting plate unit 200 is arranged at the contact surface 101 of the sensor, the color converting plate 200 will also be in direct contact to the skin 1000 of a user when the PPG sensor is placed onto the skin 1000 of the user.

According to an aspect of the invention, the light from the at least one light source 110 which is emitting from the contact surface 101 of the PPG sensor should preferably have a wavelength in the green/yellow range (e.g. in the area of 500 to 600 nm). This can either be achieved by a light source or a light emitting diode which is directly outputting light at this wavelength or this can be achieved by using a color converting plate unit 200 to change the color of the light from the light source to a desired color temperature. In other words, the wavelength of the light from the light source 110 is changed by the color converting plate 200.

Fig. 3 shows a schematic representation of an optical vital signs sensor according to a further aspect of the invention. The PPG sensor according to Fig. 3 substantially corresponds to the PPG sensor according to Fig. 2 with a coating 210 on top of the color converting plate 200 and optionally with a diffusing chamber 220. The coating or layer 210 on top of the color converting plate 200 can be implemented as an angle selective film which transmits light at small angles of incidence while reflecting light at large angles of incidence. The angle selective film 210 may comprise a multi-layer thin film interference filter like a dielectric mirror.

The optional diffusing chamber 220 can optionally be arranged around the light source or light emitting diode 110 and is used to recycle light as shown in Fig. 3.

The light source 110 emits light and a part of this light 103 passes through the color converting plate 200 and the angle selective film 210. Other parts of this light with different angles of incidence 104 are reflected from the angle selective film or coating 110. In addition, further light 105 can be recycled by the diffusion chamber 220 and can be redirected towards the color converting plate unit 200 with a different angle.

Fig. 4 shows a schematic representation of an optical vital signs sensor according to a further aspect of the invention. The PPG sensor according to Fig. 4 substantially corresponds to the PPG sensor according to Fig. 4 with a diffusion chamber 220 around the light source 110 as well as a long wave pass filter LWPF 230 on top of the color converting plate 200. The long-wave pass filter 230 can comprise a dielectric multi-layer stack which allows long wave like green/yellow light to be transmitted while reflecting short waves like blue light. According to this aspect of the invention, the long-wave pass filter coating 230 on top of the color converting plate 200 is part of the contact surface 101 of the PPG sensor such that the coating 230 is in direct contact with the skin of a user.

A part 103 of the light from the light source 110 passes through the color converting plate 200 and the long-wave pass filter 230 and enters the skin 1000 of a user. A further part 104a is reflected by the low-wave pass filter 230 and can be recycled 105a by the diffusion chamber 220.

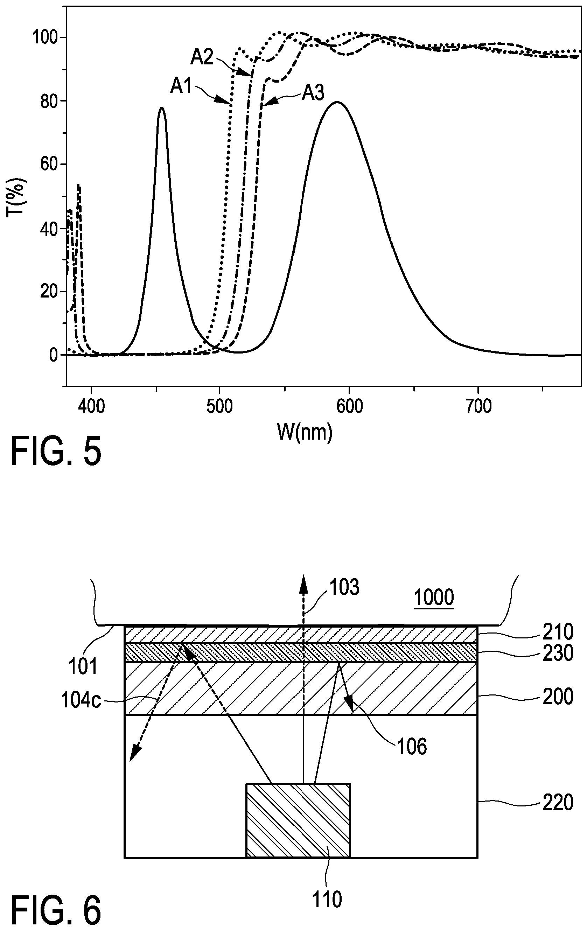

Fig. 5 shows a graph indicating the function of the transmittance over the wavelength of the optical vital signs sensor according to Fig. 4. In Fig. 5, a long-wave pass filter with a blue spectrum as well as a down-converted yellow spectrum A1, A2, A3 is depicted.

Fig. 6 shows a basic representation of part of an optical vital signs sensor according to a further aspect of the invention. The aspect of the invention according to Fig. 6 is a combination of the PPG sensor of Fig. 3 and 4. Accordingly, a light source 110 is optionally surrounded by a diffusing chamber 220 and a color converting plate 200. On top of the color converting plate 200, a long wave pass filter coating 230 is provided. On top of this long wave pass filter coating 230, an angle selective filter coating 210 is provided. With the PPG sensor according to this aspect of the invention, only green/yellow light 103 at small angles is transmitted through the two coatings 210, 230 while unconverted light (i.e. short wavelength pump- light104c) is reflected by the long-wave pass filter coating 230. Furthermore, converted light 106 which still has large angles of incidence is reflected by the angle selective filter coating 210. Once again, the diffusion chamber 220 can be used to recycle light.

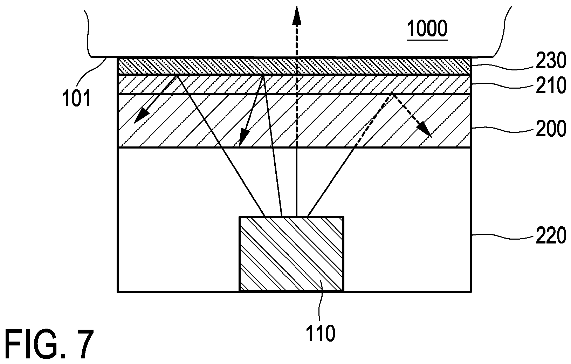

Fig. 7 shows a basic representation of a part of an optical vital signs sensor according to a further aspect of the invention. The PPG sensor according to this aspect sub stantially corresponds to the PPG sensor according to Fig. 6, wherein the order of the angle selective for a film coating 210 and the long wave pass filter coating 230 is changed.

According to the invention, the PPG sensor according to Fig. 6 appears to be more effective than the PPG sensor according to Fig. 7. This is due to the fact that the angle selective film coating 210 is designed for a narrow wavelength range. On the other hand, with the PPG sensor according to Fig. 7, the long wave pass filter coating 230 is able to reflect unconverted light at large angles of incidences.

To further reduce the optical losses at the interface between the PPG sensor and the skin of the user, optical coupling material such as a gel, liquid or oil can be provided at the contact surface 101 of the PPG sensor.

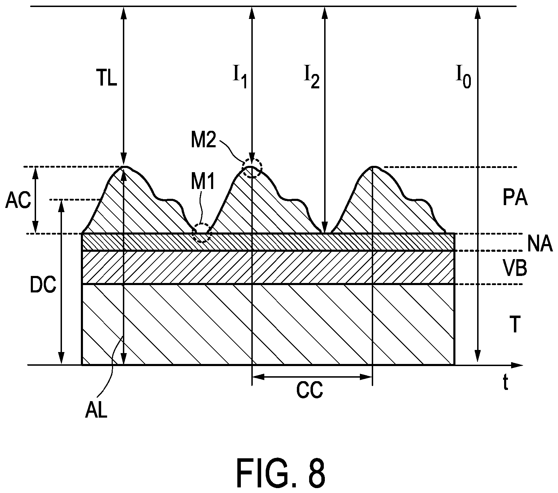

Fig. 8 shows a graph for illustrating a relative DC power and AC/DC signal of an optical vital signs sensor according to an aspect of the invention. In Fig. 8, one important property of the output signal, namely the modulation signal is depicted.

The modulation signal relates to the ratio of the AC component to the DC component. The modulation AC/DC signal is important, because it is related to intrinsic properties of the skin. It covers the peek-to-peek value of the change in blood volume fraction in one heart pulse (AC signal), but also the skin-dependent reflectance (DCcomponent DC) which is important to know because a low reflectance can be compensated with LED power boost, preserving the same modulation signal.

In Fig. 8, the output signal of the PPG sensor is depicted. Furthermore, the influence of different parts of the body, namely pulsating arterial blood PA, non-pulsating arterial blood NA, venous blood VB and other tissue is depicted. Moreover, incident light $I_0$ as well as transmitted light TL and absorbed light AL is depicted. It should be noted that according to the invention, the AC signal AC represents the component that contains the information which the sensor requires in order to determine a heart rate of a user. In other words, the AC signal represents the information regarding the pulsating arterial blood, i.e.the change in the blood volume while the DC component of the output signal represents the unwanted background signal, namely the influence of the other tissue, the venous blood VB and the non-pulsating arterial blood NA. The DC component can have 0 Hz or may also have a low frequency component which can be caused by leakage light shunted from the light source to the light detector without passing through the skin or tissue of the user (static), a dynamic variation of leakage light caused by motion (dynamic) and light detected by the detector which is reflected by the tissue or skin of the user or other matter like the venous blood VB, fat, bone, water, cell membranes, etc.

Typically, in a PPG sensor, the AC component of the output signal is smaller than the DC component. Hence, in order to obtain a good output signal, the DC component should be minimized while the AC component should be maximized in order to achieve a maximum modulation signal.

In Fig. 8, two measurements, namely M1 and M2 are depicted, wherein the first measurement M1 is measured at the minimum value of the output signal while the second value is measured at the maximum output signal.

The modulation signal can be expressed by the following equation:

$$\frac{AC}{DC} = 2 \cdot \frac{(M2 - M1)}{(M2 + M1)}.$$

It should further be noted that the modulation signal, i.e. the AC/DC signal is sensitive towards the beam pattern and the angle of incidence. The greater the distance between the light source and the photodiode, the lower the sensitivity regarding the angle of incidence. Furthermore, according to an aspect of the invention, an angle of incidence of greater than 45° should be avoided while small beam angles around 0° and a beam angle pointing in the opposite direction as towards the photodiode can also be used. According to an aspect of the invention, an improved PPG signal can be obtained if the beam angle of the light source is $< \pm 20°$.

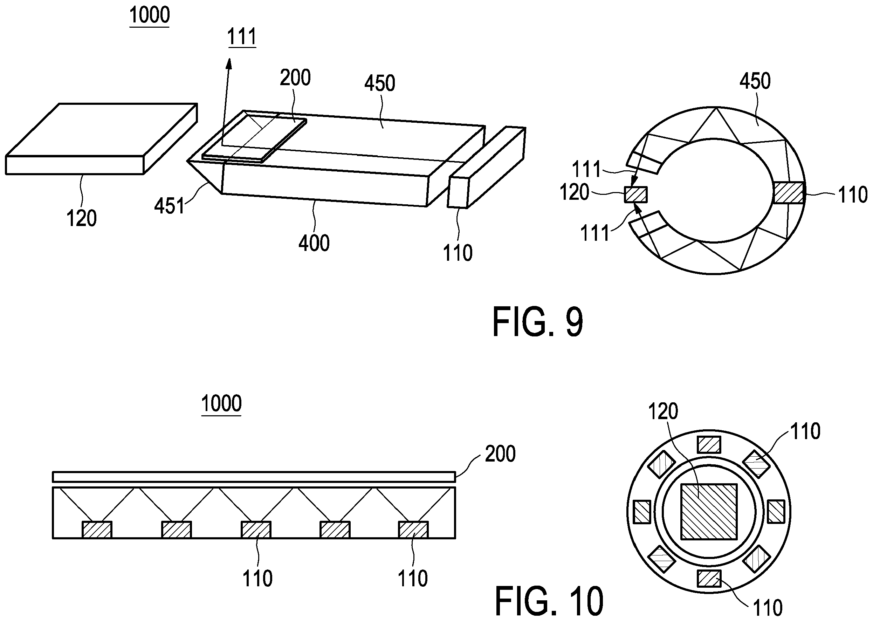

Fig. 9 shows a basic representation of a vital signs sensor.

The vital signs sensor according to Fig. 9 comprises at least one light source 110, at least one photodiode 120 as well as at least one light guide 400. According to this aspect of the invention, the light guide 400 is arranged between the at least one light source 110 and the at least one photodiode 120. The light guide 400 is implemented as a light transport unit 450 which is able to transport light from the at least one light source (for example a LED which is implemented as a side emitter) towards the at least one photodiode 120. The distal end of the light transport unit 450 has an inclination 451 such that the light 111 from the at least one light source 110 is redirected towards the skin of the user 1000. With such a light guide unit 400, the distance between the photodiode 120 and the output end of the light guide unit 450 can be significantly reduced and a flat design with a low building height is possible. Furthermore, a color converting plate 200 as described above can be provided between the light guide 400 and the skin 1000 of a user.

Fig. 10 shows a basic representation of a vital signs sensor. The vital signs sensor can comprise at least one light unit 110, a photo detector 120 and an optical angle selective foil 200. The angle selective optical foil as color converting plate unit 200 is able to allow light to transmit within a selected angle range. Alternatively, the color converting unit can also be implemented with an optical holographic light shaping diffuser or direction turning film DTF.

The color converting unit 200 is used to shape, direct, redirect, control or manage the light beam from the light source such that the angular range of the beam is limited or restricted.

Other variations can be understood and effected by those skilled in the art from a study of the drawings, the disclosure and the appended claims.

In the claims, the word "comprising" does not exclude other elements or steps and in the indefinite article "a" or "an" does not exclude a plurality.

A single unit or device may fulfill the functions of several items The mere fact that certain measures are recited in mutual different dependent claims does not indicate that a combination of these measurements cannot be used to advantage. A computer program may be stored/distributed on a suitable medium such as an optical storage medium or a solid state medium, supplied together with or as a part of other hardware, but may also be distributed in other forms such as via the internet or other wired or wireless telecommunication systems.

Any reference signs in the claims should not be construed as limiting the scope. The invention is defined in the appended claims.

**Claims**

1. Optical vital signs sensor (100) configured to measure or determine vital signs of a user, comprising:

   - a contact surface (101) configured to be placed directly against a skin (1000) of a user,
   - at least one color converting plate unit (200) arranged in or at the contact surface (101),
   - at least one light source (110) configured to generate light which is directed towards a skin (1000) of the user via the at least one color converting plate unit (200), wherein said at least one color converting plate unit (200) is configured to change the color of the light from the at least one light source (110) to a desired color temperature,
   - at least one photo detector unit (120) configured to detect light which is indicative of a reflection of the light emitted via the at least one color converting plate (200) in or from the skin (1000) of the user, and

   wherein the color converting plate unit (200) comprises: an angle selective optical coating (210) which is able to reflect or redirect light having a large angle of incidence and to transmit light having a small angle of incidence, and a diffusing chamber (220) arranged around the at least one light source (110) and being configured to recycle light by redirecting light towards the color converting plate unit (200) with a different angle.

2. Optical vital signs sensor (100) according to claim 1, wherein the color converting plate (200) comprises a long-wave pass filter coating which is able to transmit light having a long wavelength while reflecting light having short wavelengths.

3. Optical vital signs sensor (100) according to claim 2, wherein the at least one light source (110) comprises an InGaN light emitting diode.

4. Optical vital signs sensor (100) according to claim 3, wherein the at least one color converting plate unit (200) is configured to convert the light from the In-GaN light emitting diode to green or yellow light having approximately a wavelength of 500 to 600 nm.

5. Wearable device comprising at least one optical vital signs sensor according to one of the claims 1 to 4.

6. Method of operating an optical vital signs sensor (100) according to one of the claims 1 to 4, comprising the steps of:

- placing the contact surface (101) of the optical vital signs sensor (100) directly against a skin (1000) of a user, the least one color converting plate unit (200) being arranged in or at the contact surface (101),
- generating light by the at least one light source (110) and directing the light towards a skin (1000) of a user via the at least one color converting plate unit (200) and
- detecting light which is indicative of a reflection of the light emitted via the at least one color converting plate (200) in or from the skin (1000) of the user, by the at least one photo detector unit (120),
- recycling light of one of the at least one light sources (110) by redirecting it towards the color converting plate unit at a different angle by the diffusing chamber (220) around the at least one light source (110).

**Patentansprüche**

1. Optischer Vitalparametersensor (100) zum Messen oder Ermitteln der Vitalparameter eines Benutzers, der Folgendes umfasst:

- eine Kontaktfläche (101), die direkt auf der Haut (1000) des Benutzers angebracht werden kann,
- mindestens eine Farbumwandlungsplatten-Einheit (200), die sich in oder an der Kontaktfläche (101) befindet,
- mindestens eine Lichtquelle (110), die Licht erzeugt, das über die mindestens eine Farbumwandlungsplatten-Einheit (200) auf die Haut (1000) des Benutzers gerichtet wird,

wobei die mindestens eine Farbumwandlungsplatten-Einheit (200) die Farbe des Lichts der mindestens einen Lichtquelle (110) in die gewünschte Farbtemperatur ändert,

- mindestens eine Fotodetektoreinheit (120), die Licht erkennt, das auf eine Reflexion des über die mindestens eine Farbumwandlungsplatte (200) emittierten Lichts in oder von der Haut (1000) des Benutzers hinweist, und

wobei die Farbumwandlungsplatten-Einheit (200) Folgendes umfasst: eine winkelselektive optische Beschichtung (210), die Licht mit einem großen Einfallswinkel reflektieren oder umlenken sowie Licht mit einem kleinen Einfallswinkel durchlassen kann, und
eine Diffusionskammer (220), die um die mindestens eine Lichtquelle (110) herum angeordnet und so konfiguriert ist, dass sie das Licht wiederverwertet, indem sie das Licht in einem anderen Winkel auf die Farbumwandlungsplatten-Einheit (200) umleitet.

2. Optischer Vitalparametersensor (100) gemäß Anspruch 1, wobei die Farbumwandlungsplatte (200) eine Langwellen-Passfilterbeschichtung umfasst, die Licht mit einer langen Wellenlänge durchlassen kann, während sie Licht mit kurzer Wellenlänge reflektiert.

3. Optischer Vitalparametersensor (100) gemäß Anspruch 2, wobei die mindestens eine Lichtquelle (110) eine InGaN-Leuchtdiode umfasst.

4. Optischer Vitalparametersensor (100) gemäß Anspruch 3, wobei die mindestens eine Farbumwandlungsplatten-Einheit (200) das Licht der InGaN-Leuchtdiode in grünes oder gelbes Licht mit einer Wellenlänge von etwa 500 bis 600 nm umwandelt.

5. Tragbares Gerät mit mindestens einem optischen Vitalparametersensor gemäß einem der Ansprüche 1 bis 4.

6. Methode zum Betreiben eines optischen Vitalparametersensors (100) gemäß einem der Ansprüche 1 bis 4,
die folgende Schritte umfasst:

- Platzieren der Kontaktfläche (101) des optischen Vitalparametersensors (100) direkt auf der Haut (1000) des Benutzers,

wobei sich die mindestens eine Farbumwandlungsplatten-Einheit (200) in oder an der Kontaktfläche (101) befindet,

- Generieren von Licht mit der mindestens einen Lichtquelle (110) und Richten des Lichts auf die Haut (1000) des Benutzers über die mindestens eine Farbumwandlungsplatten-Einheit (200) und
- Erkennen von Licht, das auf eine Reflexion des über die mindestens eine Farbumwandlungsplatte (200) emittierten Lichts in oder von der Haut (1000) des Benutzers hinweist, mithilfe mindestens einer Fotodetektoreinheit (120),
- Wiederverwerten des Lichts der mindestens einen Lichtquelle (110), indem es durch die Diffusionskammer (220) um die mindestens eine Lichtquelle (110) herum in einem anderen Winkel auf die Farbumwandlungsplatten-Einheit umgeleitet wird.

**Revendications**

1. Capteur optique (100) de signes vitaux conçu pour mesurer ou pour déterminer les signes vitaux d'un utilisateur, comprenant :

- une surface de contact (101) conçue pour être placée directement sur une peau (1000) d'un utilisateur,
- au moins une unité de plaque (200) de conversion de couleur disposée dans ou au niveau de la surface de contact (101),
- au moins une source lumineuse (110) conçue pour générer de la lumière, laquelle est dirigée vers une peau (1000) de l'utilisateur par l'intermédiaire de l'au moins une unité de plaque (200) de conversion de couleur, dans lequel ladite au moins une unité de plaque (200) de conversion de couleur est conçue pour changer la couleur de la lumière provenant de l'au moins une source lumineuse (110) à une température chromatique souhaitée,
- au moins une unité de photodétecteur (120) conçue pour détecter une lumière, laquelle indique une réflexion de la lumière émise par l'intermédiaire de l'au moins une plaque (200) de conversion de couleur dans ou à partir de la peau (1000) de l'utilisateur, et
dans lequel l'unité de plaque (200) de conversion de couleur comprend : un revêtement optique (210) sélectif d'angle, lequel est capable de réfléchir ou de rediriger la lumière présentant un grand angle d'incidence et de transmettre la lumière présentant un petit angle d'incidence, et une chambre de diffusion (220) disposée autour de l'au moins une source lumineuse (110) et étant conçue pour recycler la lumière par redirection de la lumière vers l'unité de plaque (200) de conversion de couleur à un angle différent.

2. Capteur optique (100) de signes vitaux selon la revendication 1, dans lequel la plaque (200) de conversion de couleur comprend un revêtement de filtre passe-ondes, lequel est capable de transmettre une lumière présentant une longue longueur d'onde tout en réfléchissant la lumière présentant de courtes longueurs d'onde.

3. Capteur optique (100) de signes vitaux selon la revendication 2, dans lequel l'au moins une source lumineuse (110) comprend une diode électroluminescente InGaN.

4. Capteur optique (100) de signes vitaux selon la revendication 3, dans lequel l'au moins une unité de plaque (200) de conversion de couleur est conçue pour convertir la lumière provenant de la diode électroluminescente InGaN en lumière verte ou jaune présentant approximativement une longueur d'onde comprise entre 500 et 600 nm.

5. Dispositif portable comprenant au moins un capteur optique de signes vitaux selon l'une quelconque des revendications 1 à 4.

6. Procédé de fonctionnement d'un capteur optique (100) de signes vitaux selon l'une quelconque des revendications 1 à 4, comprenant les étapes suivantes :

- le placement de la surface de contact (101) du capteur optique (100) de signes vitaux directement sur une peau (1000) d'un utilisateur, l'au moins une unité de plaque (200) de conversion de couleur étant disposée dans ou au niveau de la surface de contact (101),
- la génération de la lumière par l'au moins une source lumineuse (110) et la direction de la lumière vers une peau (1000) d'un utilisateur par l'intermédiaire de l'au moins une unité de plaque (200) de conversion de couleur, et
- la détection d'une lumière, laquelle indique une réflexion de la lumière émise par l'intermédiaire de l'au moins une plaque (200) de conversion de couleur dans ou à partir de la peau (1000) de l'utilisateur, par l'au moins une unité de photodétecteur (120),
- le recyclage hermétique de l'une des au moins une source lumineuse (110), par redirection de celle-ci vers l'unité de plaque de conversion de couleur sous un angle différent, par la chambre de diffusion (220) autour de l'au moins une source lumineuse (110).

FIG. 1

FIG. 2

FIG. 3

FIG. 4

FIG. 5

FIG. 6

1000

230
210
200

101

220

110

FIG. 7

FIG. 8

FIG. 9

FIG. 10

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2006110488 A2
- US 20120078116 A1
- EP 2139383 B1
- JP 2001025462 A
- US 20140243648 A1